# EUROPÄISCHE PATENTSCHRIFT

(11) **EP 0 873 333 B1**
(45) Veröffentlichungstag und Bekanntmachung des Hinweises auf die Patenterteilung: **13.02.2002**
(21) Anmeldenummer: 96934495.1
(22) Anmeldetag: 30.09.1996
(51) Int. Cl.: C07D 317/22, C07C 69/734, C07C 251/48, C07D 319/06, C07D 263/06, C07D 265/06, C07D 277/04, C07D 279/06, C07D 339/08, C07C 303/20

(54) **PHENYLBENZYLETHER, VERFAHREN ZU IHRER HERSTELLUNG UND IHRE VERWENDUNG ALS SCHÄDLINGSBEKÄMPFUNGSMITTEL UND FUNGIZID**
PHENYL BENZYL ETHERS, PROCESS FOR PRODUCING THEM AND THEIR USE AS PESTICIDE AND FUNGICIDE
ETHERS PHENYLBENZYLIQUES, LEUR PROCEDE DE PRODUCTION ET LEUR UTILISATION COMME PESTICIDE ET FONGICIDE

(30) Priorität: 19.10.1995 DE 19538855
(43) Veröffentlichungstag der Anmeldung: 28.10.1998
(73) Patentinhaber: BASF AKTIENGESELLSCHAFT, 67056 Ludwigshafen (DE)
(72) Erfinder: SAUTER, Hubert, D-68167 Mannheim (DE); GRAMMENOS, Wassilios, D-67063 Ludwigshafen (DE); MÜLLER, Bernd, D-67227 Frankenthal (DE); OBERDORF, Klaus, D-69117 Heidelberg (DE); GÖTZ, Norbert, D-67547 Worms (DE); RÖHL, Franz, D-67105 Schifferstadt (DE); AMMERMANN, Eberhard, D-64646 Heppenheim (DE); LORENZ, Gisela, D-67434 Hambach (DE); STRATHMANN, Siegfried, D-67117 Limburgerhof (DE)
(86) Internationale Anmeldenummer: EP9604263
(87) Internationale Veröffentlichungsnummer: WO9714693

(56) Entgegenhaltungen:
- EP-A- 0 386 561
- EP-A- 0 400 417
- EP-A- 0 513 580
- DE-A- 4 312 637

## Beschreibung

Die vorliegende Erfindung betrifft Verbindungen der allgemeinen Formel I 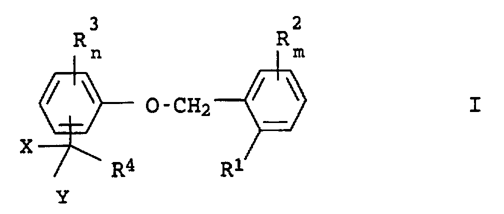 in der die Indices und die Substituenten die folgende Bedeutung haben:
- R¹: C (CO₂CH₃) =CHOCH₃, C (CO₂CH₃) =NOCH₃, C (CONHCH₃) =NOCH₃ und N(OCH₃) -CO₂CH₃;
- R²: Cyano, Halogen, C₁-C₄-Alkyl und C₁-C₄-Alkoxy;
- m: 0 oder 1;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- X,Y: C₁-C₆-Alkoxy, C₁-C₆-Alkylthio, C₁-C₆-Alkylamino, Di-C₁-C₆-alkylamino oder gemeinsam eine C₁-C₄-Alkylenkette, welche über Sauerstoff-, Schwefel- und/oder Stickstoff an das C-Atom gebunden ist und welche einen oder zwei der folgenden Substituenten tragen kann: Oxo (=O), Cyano, C₁-C₄-Alkyl, C₃-C₆-Cycloalkyl, C₁-C₄-Alkoxycarbonyl, Aryl und Heteroaryl.

Außerdem betrifft die Erfindung Verfahren zur Herstellung dieser Verbindungen, sie enthaltende Mittel und deren Verwendung zur Bekämpfung von Schädlingen oder Schadpilzen.

Aus der Literatur sind Ketone bzw. Aldehyde der Phenylbenzylether als Zwischenprodukte bekannt (EP-A 513 580; DE-A 43 12 637; EP-A-386561). Durch Cycloalkyl substituierte Phenylbenzylether sind aus EP-A-400417 bekannt.

Der Erfindung lagen demgegenüber neue Wirkstoffe mit fungiziden und pestiziden Eigenschaften als Aufgabe zugrunde.

Demgemäß wurden die eingangs definierten Verbindungen I gefunden. Außerdem wurden Verfahren zu ihrer Herstellung, sie enthaltende Mittel und deren Verwendung zur Bekämpfung von Schädlingen oder Schadpilzen gefunden.

Die Herstellung der Verbindungen I erfolgt in Analogie zu aus der Literatur bekannten Methoden.

Beispielsweise erhält man Verbindungen I, in denen die Reste X und Y gemeinsam mit dem C-Atom, an das sie gebunden sind, für einen Oxiranring stehen, in an sich bekannter Weise [Reactions of Organosulfur Compounds, S. 101-105, Academic Press, New York 1978; Adv. Org. Chem. 6, 285-388 (1969)] dadurch, daß man eine Carbonylverbindung der Formel II in einem inerten organischen Lösungsmittel in Gegenwart einer Base mit einem Trimethylsulfoxonium- oder Trimethylsulfonium Halogenid umsetzt.

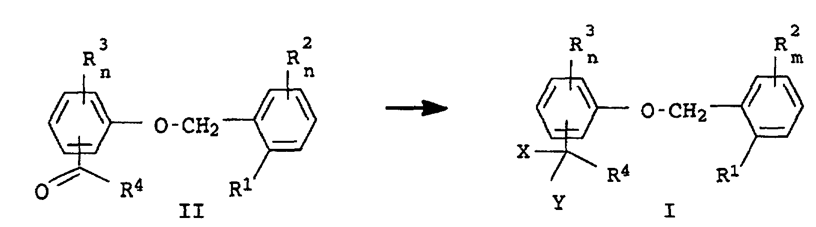

Diese Umsetzung erfolgt üblicherweise bei Temperaturen von 0°C bis 150°C, vorzugsweise 20°C bis 120°C.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, sowie Dimethylsulfoxid und Dimethylformamid, besonders bevorzugt Dimethylsulfoxid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetallhydride wie Lithiumhydrid, Natriumhydrid, Kaliumhydrid und Calciumhydrid, metallorganische Verbindungen, insbesondere Alkalimetallalkyle wie Methyllithium, Butyllithium und Phenyllithium, Alkalimetall- und Erdalkalimetallalkoholate wie Natriummethanolat, Natriumethanolat, Kaliumethanolat, Kalium- tert.-Butanolat und Dimethoxymagnesium in Betracht. Besonders bevorzugt werden Natriummethylat, Natriumhydrid, Natriumhydroxid, Kaliumhydroxid und Kalium-tert.-butylat.

Die Basen werden im allgemeinen in katalytischen Mengen eingesetzt, sie können aber auch äquimolar, im Überschuß oder gegebenenfalls als Lösungsmittel verwendet werden.

Die für die Herstellung der Verbindungen I benötigten Carbonylverbindungen sind in der eingangs zitierten Literatur beschrieben.

Verbindungen I, in denen die Reste X und Y über Sauerstoff-, Schwefel- oder Stickstoff an das C-Atom gebunden sind und R¹ für C[CONHCH₃]=NOCH₃ steht, erhält man beispielsweise dadurch, daß man eine Carbonylverbindung der Formel II in an sich bekannter Weise [Synthesis 501-522 (1981), Synthesis 203 (1983); Carboxylic Ortho Ester Derivatives, S. 154-164, Academic Press, New York 1970] in einem inerten organischen Lösungsmittel sowie gegebenenfalls einer Säure und/oder eines sauren Katalysators entweder a) mit einem Alkohol, Thiol und/oder Amin der Formel III oder b) einer Verbindung der Formel IV umsetzt.

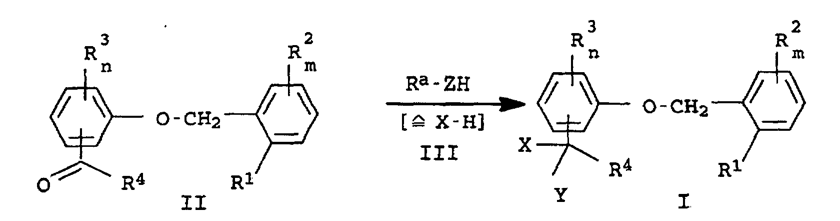

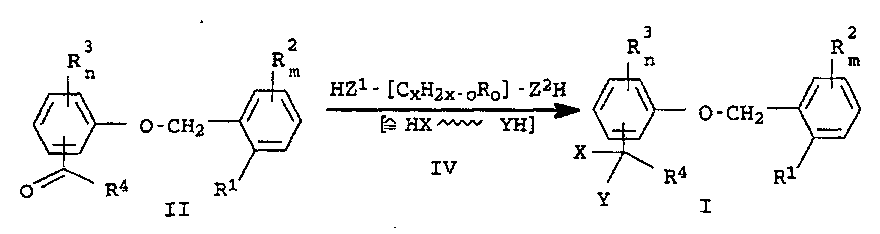

In der Formel III, die allgemein Verbindungen der Formeln XH bzw. YH repräsentiert, bedeutet R^{a} eine C₁-C₆-Alkylgruppe. Z in der Formel III bedeuten Sauerstoff, Schwefel, Amino (NH) oder C₁-C₆-Alkylamino und Z¹ und Z² in der Formel IV bedeuten Sauerstoff, Schwefel oder Amino (NH).

Die Formel IV steht repräsentativ für Vorprodukte, bei denen X und Y für eine C₁-C₄-Alkylenkette stehen, welche über Sauerstoff, Schwefel und/oder Stickstoff an das C-Atom gebunden ist. Demgemäß steht x für 1, 2, 3 oder 4 und o für 0, 1, 2 oder 3. R bedeutet C₁-C₄-Alkyl, C₃-C₆-Cycloalkyl, C₁-C₄-Alkoxycarbonyl, Cyano, Aryl und Heteroaryl, oder eine oder mehrere CH₂-Gruppen der Alkylenkette können durch C=O ersetzt sein.

Diese Umsetzung erfolgt üblicherweise bei Temperaturen von 0°C bis 180°C, vorzugsweise 20°C bis 150°C.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, sowie Dimethylsulfoxid und Dimethylformamid, besonders bevorzugt Toluol, Methylenchlorid, Chlorbenzol und Xylole.

Es können auch Gemische der genannten Lösungsmittel verwendet werden.

Als Säuren und saure Katalysatoren können anorganische Säuren wie Fluorwasserstoffsäure, Salzsäure, Bromwasserstoffsäure, Schwefelsäure und Perchlorsäure, Lewis-Säuren wie Bortrifluorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure, Trifluormethansulfonsäure, Methansulfonsäure und Toluolsulfonsäure Verwendung finden.

Die Säuren bzw. sauren Katalysatoren werden im allgemeinen in katalytischen Mengen eingesetzt, sie können aber auch äquimolar, im Überschuß oder gegebenenfalls als Lösungsmittel verwendet werden.

Verbindungen I, in denen R^{a} nicht Methyl bedeutet, erhält man besonders vorteilhaft in analoger Weise dadurch, daß man die Carbonylverbindung II zunächst mit Ameisensäuretrimethylester oder Methanol in das entsprechende Dimethylacetal bzw. -ketal (I, X = Y = OCH₃) überführt und dieses anschließend entweder mit einer Verbindung III oder einer Verbindung IV umacetalisiert.

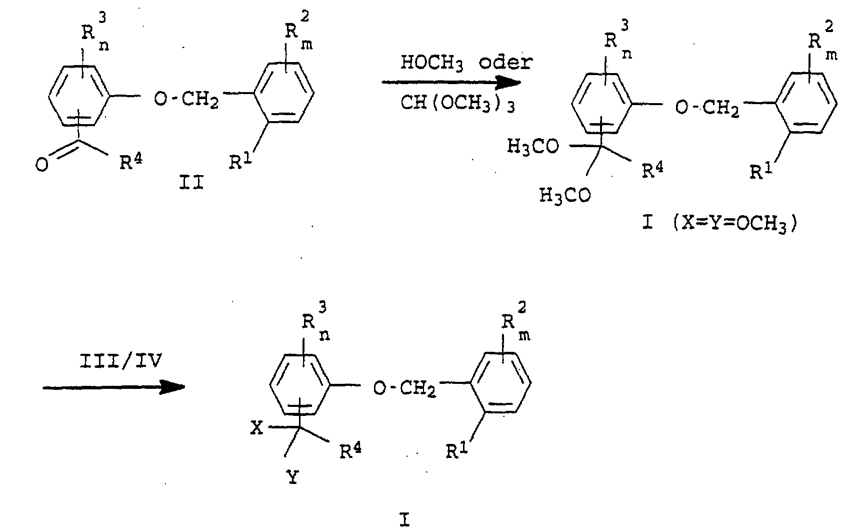

Diese Umsetzung von II mit Ameisensäureorthomethylester erfolgt üblicherweise bei Temperaturen von 0°C bis 180°C, vorzugsweise 20°C bis 150°C.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, sowie Dimethylsulfoxid und Dimethylformamid, besonders bevorzugt Dimethylsulfoxid und Dimethylformamid. Es können auch Gemische der genannten Lösungsmittel verwendet werden.

Als Säuren und saure Katalysatoren können anorganische Säuren wie Fluorwasserstoffsäure, Salzsäure, Bromwasserstoffsäure, Schwefelsäure und Perchlorsäure, Lewis-Säuren wie Bortrifluorid, Zinn-IV-chlorid und Zinn-II-chlorid, sowie organische Säuren wie Ameisensäure, Essigsäure, Propionsäure, Oxalsäure, Zitronensäure, Trifluoressigsäure, Methansulfonsäure und 4-Methylphenylsulfonsäure Verwendung finden.

Die Säuren bzw. saure Katalysatoren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, Ameisensäureorthomethylester in einem Über- oder Unterschuß, bezogen auf II, einzusetzen.

Nach einem weiteren Verfahren erhält man die Verbindungen I auch dadurch, daß man ein Phenol der Formel V in an sich bekannter Weise in einem inerten organischen Lösungsmittel in Gegenwart einer Base mit einer Benzylverbindung der Formel VI umsetzt.

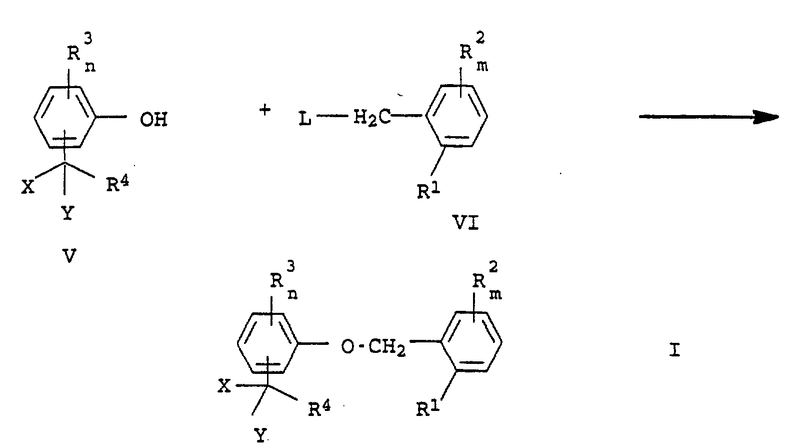

L in der Formel VI steht für eine nucleophil austauschbare Gruppe wie Halogen (Fluor, Chlor, Brom und Iod, vorzugsweise Chlor, Brom und Iod) oder eine Alkyl- oder Arylsulfonatgruppe (z.B. Methylsulfonat, Trifluormethylsulfonat, Phenylsulfonat und 4-Methylphenylsulfonat).

Diese Umsetzung erfolgt üblicherweise bei Temperaturen von 0°C bis 120°C, vorzugsweise 20°C bis 85°C.

Geeignete Lösungsmittel sind Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon sowie Dimethylsulfoxid und Dimethylformamid, besonders bevorzugt Tetrahydrofuran, Aceton, Acetonitril, Dimethylsulfoxid und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat Natriumcarbonat, Kaliumcarbonat und Calciumcarbonat sowie Alkalimetallhydrogencarbonate wie Natriumhydrogencarbonat, sowie Alkalimetall- und Erdalkalimetallalkoholate wie Natriummethanolat, Natriumethanolat, Kaliumethanolat, Kalium-tert.-butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydrid, Kaliumcarbonat, Natriummethanolat und Triethylamin.

Die Basen werden im allgemeinen in katalytischen Mengen eingesetzt, sie können aber auch äquimolar, im Überschuß oder gegebenenfalls als Lösungsmittel verwendet werden.

Die für die Herstellung der Verbindungen I benötigten Ausgangsstoffe sind aus der Literatur bekannt (EP 420 091 A1; EP 38 65 561 A1; DE 36 20 860 A1; DE 35 45 318 A1; WO 93/15046 A1) oder können gemäß der zitierten Literatur hergestellt werden.

Verbindungen Va, in denen die Reste X und Y über Sauerstoff-, Schwefel- oder Stickstoff an das C-Atom gebunden sind, erhält man besonders vorteilhaft dadurch, daß man einen Phenolester der Formel VII in an sich bekannter Weise in einem inerten organischnen Lösungsmittel mit a) einem Alkohol, Thiol und/oder Amin der Formel III oder mit b) einer Verbindung der Formel IV (oder wie vorstehend beschrieben, zunächst mit Ameisensäureorthomethylester oder Methanol in das entsprechende Dimethylacetal bzw. -ketal (VIII, X = Y = OCH₃) überführt und dieses anschließend entweder mit einer Verbindung III oder einer Verbindung IV umacetalisiert in den entsprechenden Phenolester VIII überführt und VIII anschließend in Gegenwart einer Base zu V spaltet.

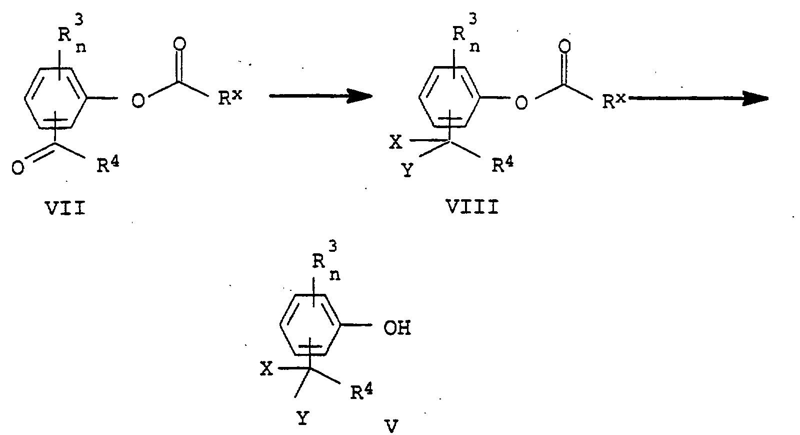

R^{x} in den Formeln VII und VIII steht für Phenyl oder eine Alkylgruppe (z.B. C₁-C₄-Alkyl).

Diese Esterspaltung erfolgt üblicherweise bei Temperaturen von 0°C bis 120°C, vorzugsweise 0°C bis 80°C.

Geeignete Lösungsmittel sind neben Wasser Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol sowie Dimethylsulfoxid und Dimethylformamid, besonders bevorzugt Wasser, Methanol, Dimethylformamid, Dimethylsulfoxid und Tetrahydrofuran. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetallamide wie Lithiumamid, Natriumamid und Kaliumamid, Alkalimetall- und Erdalkalimetallcarbonate wie Natriumcarbonat, Kaliumcarbonat, Calciumcarbonat, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumamid, Natriumhydroxid, Kaliumhydroxid und Natriummethanolat.

Die Basen werden im allgemeinen in katalytischen Mengen eingesetzt, sie können aber auch äquimolar, im Überschuß oder gegebenenfalls als Lösungsmittel verwendet werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z. T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Verbindungen I nach dem vorstehend beschriebenen Verfahren benötigten Ausgangsstoffe sind in der Literatur bekannt (Polym. Commun. 337 (1984); EP 353 339 A2) oder können gemäß der zitierten Literatur hergestellt werden.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 6 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 6 Kohlenstoffatomen (wie vorstehend genannt), welche über ein Sauerstoffatom (-O-) an das Gerüst gebunden sind;
Alkylthio: geradkettige oder verzweigte Alkylgruppen mit 1 bis 6 Kohlenstoffatomen (wie vorstehend genannt), welche über ein Schwefelatom (-S-) an das Gerüst gebunden sind;
Alkylamino: eine geradkettige oder verzweigte Alkylgruppe mit 1 bis 6 Kohlenstoffatomen (wie vorstehend genannt), welche über eine Aminogruppe (-NH-) an das Gerüst gebunden ist;
Dialkylamino: eine Aminogruppe, welche zwei voneinander unabhängige, geradkettige oder verzweigte Alkylgruppen mit jeweils 1 bis 6 Kohlenstoffatomen (wie vorstehend genannt) trägt;
Alkoxycarbonyl: geradkettige oder verzweigte Alkylgruppen mit 1 bis 4 Kohlenstoffatomen (wie vorstehend genannt), welche über eine Oxycarbonylgruppe (-O-CO-) an das Gerüst gebunden sind;
Cycloalkyl: monocyclische Alkylgruppen mit 3 bis 6 Kohlenstoffringgliedern, z.B. wie Cyclopropyl, Cyclobutyl, Cyclopentyl und Cyclohexyl;
Aryl: mono- oder polycyclische aromatische Kohlenwasserstoffe wie Phenyl, Naphthyl und Anthracenyl;

Heteroaryl: mono- oder polycyclische aromatische Ringsysteme, welche neben Kohlenstoffatomen Stickstoff-, Sauerstoff- und/oder Schwefelatome als Ringglieder enthalten können, z.B.
- **5-gliedriges Heteroaryl, enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol -3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
- **benzokondensiertes 5-gliedriges Heteroaryl, enthaltend ein bis drei Stickstoffatome oder ein Stickstoffatom und ein Sauerstoff- oder Schwefelatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
- **über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
- **6-gliedriges Heteroaryl, enthaltend ein bis drei bzw. ein bis vier Stickstoffatome:** 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Insbesondere werden Verbindungen I bevorzugt, in denen R¹ für C(CO₂CH₃)=CHOCH₃ steht.

Daneben werden Verbindungen I bevorzugt, in denen R¹ für C(CO₂CH₃)=NOCH₃ steht.

Gleichermaßen werden Verbindungen I bevorzugt, in denen R¹ für C(CONHCH₃)=NOCH₃ steht.

Außerdem werden Verbindungen I bevorzugt, in denen R¹ für N(OCH₃)-CO₂CH₃ steht.

Insbesondere werden auch Verbindungen I bevorzugt, in denen m für 0 oder 1 steht.

Außerdem werden Verbindungen I besonders bevorzugt, in denen m für 1 steht und R² Cyano, Fluor, Chlor, Methyl oder Methoxy bedeutet.

Gleichermaßen besonders bevorzugt sind Verbindungen I, in denen n für 1 oder 2 steht.

Daneben werden Verbindungen I besonders bevorzugt, in denen R³ für Halogen oder C₁-C₄-Alkyl steht.

Besonders bevorzugt sind Verbindungen I, in denen n für 1 oder 2 steht und die Reste R³ in der 2- bzw. 2,5-Position des Phenylrings gebunden sind.

Insbesondere werden auch Verbindungen I bevorzugt, in denen der Rest -CXYR⁴ in der 4- oder 5-Position des Phenylrings steht. Außerdem werden Verbindungen I besonders bevorzugt, in denen R⁴ für Wasserstoff, Methyl, Ethyl, Trifluormethyl oder Cyclopropyl steht.

Gleichermaßen besonders bevorzugt sind Verbindungen I, in denen X für C₁-C₆-Alkoxy, C₁-C₆-Alkylthio, C₁-C₆-Alkylamino und Di-C₁-C₆-alkylamino steht.

Daneben werden Verbindungen I besonders bevorzugt, in denen Y für C₁-C₆-Alkoxy, C₁-C₆-Alkylthio, C₁-C₆-Alkylamino und Di-C₁-C₆-alkylamino steht.

Des weiteren werden Verbindungen I besonders bevorzugt, in denen X und Y gemeinsam mit dem C-Atom an das sie gebunden sind, für einen ggf. substituierten Oxiranring stehen.

Außerdem werden Verbindungen I besonders bevorzugt, in denen X und Y für eine ggf. substituierte C₂-C₄-Alkylenkette, welche über Sauerstoff-, Schwefel- und/oder Stickstoffatome an das C-Atom gebunden ist, stehen.

Gleichermaßen bevorzugt sind Verbindungen I, in denen X und Y gemeinsam mit dem C-Atom, an das sie gebunden sind, für einen der folgenden Heterocyclen XY.1 bis XY.9 stehen: 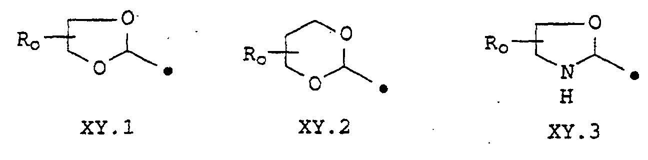 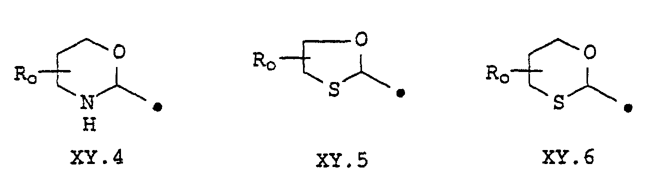 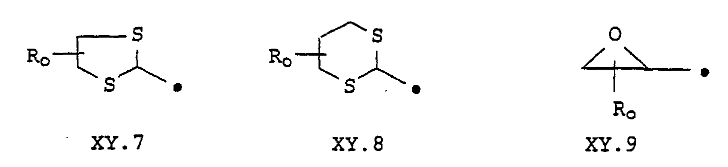 wobei die mit • gekennzeichnete Bindung die Bindung zum Phenylring repräsentiert und wobei
- o: 0, 1, 2 oder 3 bedeutet, wobei die Substituenten R verschieden sein können, wenn o für 2 oder 3 steht; und
- R: für C₁-C₄-Alkyl, C₃-C₆-Cycloalkyl, C₁-C₄-Alkoxycarbonyl, Aryl, Cyano und Heteroaryl steht oder wobei eine oder mehrere CH₂-Gruppen der Alkylenkette durch C=O ersetzt sein können.

Insbesondere werden Verbindungen der Formel I.A 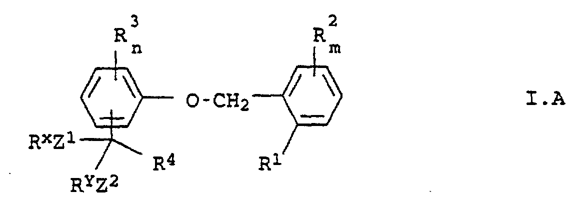 bevorzugt, in denen die Substituenten die folgende Bedeutung haben:
- R¹: C(CO₂CH₃)=CHOCH₃, C(CO₂CH₃)=NOCH₃, N(OCH₃)CO₂CH₃ und C(CONHCH₃)=NOCH₃;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- Z¹,Z²: Sauerstoff, Schwefel, Amino (MH) oder C₁-C₆-Alkylamino; und
- R^{x}, R^{y}: C₁-C₆-Alkyl.

Insbesondere werden außerdem Verbindungen der Formel I.B 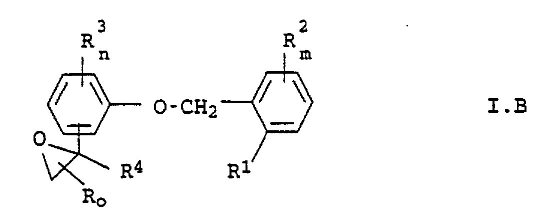 bevorzugt, in denen die Substituenten die folgende Bedeutung haben:
- R¹: C(CO₂CH₃)=CHOCH₃, C(CO₂CH₃)=NOCH₃, N(OCH₃)CO₂CH₃ und C(CONHCH₃)=NOCH₃;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0.

Insbesondere werden daneben Verbindungen der Formel I.C 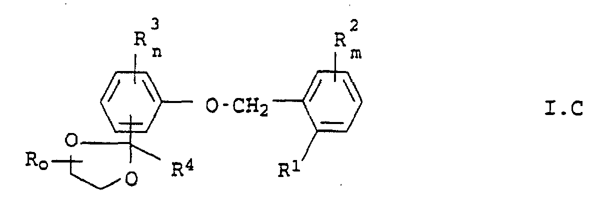 bevorzugt, in denen die Substituenten die folgende Bedeutung haben:
- R¹: C(CO₂CH₃) =CHOCH₃, C (CO₂CH₃) =NOCH₃, N(OCH₃) CO₂CH₃ und C(CONHCH₃) =NOCH₃;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0, 1, 2 oder 3, wobei die Substituenten R verschieden sein können, wenn o für 2 oder 3 steht; und
- R: C₁-C₄-Alkyl, C₃-C₆-Cycloalkyl, C₁-C₄-Alkoxycarbonyl, Cyano, Aryl und Heteroaryl oder wobei eine CH₂-Gruppe der Alkylenkette durch C=O ersetzt sein kann.

Insbesondere werden außerdem Verbindungen der Formel I.D 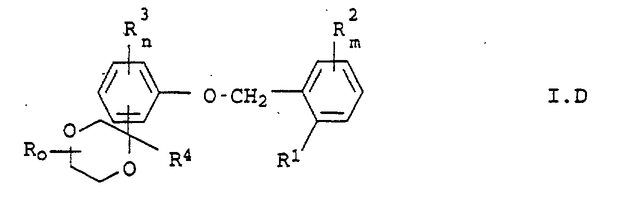 bevorzugt, in denen die Substituenten die folgende Bedeutung haben:
- R¹: C (CO₂CH₃)=CHOCH₃, C (CO₂CH₃) =NOCH₃, N (OCH₃)CO₂CH₃ und C (CONHCH₃) =NOCH₃ ;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R3: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0, 1, 2 oder 3, wobei die Substituenten R verschieden sein können, wenn o für 2 oder 3 steht; und
- R: C₁-C₄-Alkyl, C₃-C₆-Cycloalkyl, C₁-C₄-Alkoxycarbonyl, Cyano, Aryl und Heteroaryl oder wobei eine CH₂-Gruppe der Alkylenkette durch C=O ersetzt sein kann.

Insbesondere werden daneben Verbindungen der Formel I.E 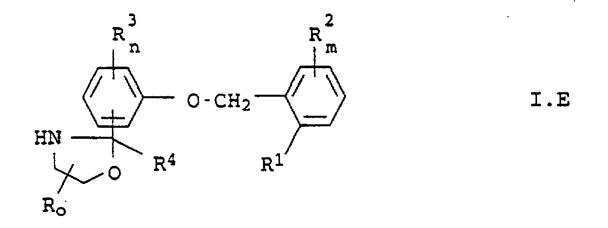 bevorzugt, in denen die Substituenten die folgende Bedeutung haben:
- R¹: C(CO₂CH₃)=CHOCH₃, C (CO₂CH₃) =NOCH₃, N(OCH₃) CO₂CH₃ und C(CONHCH₃)=NOCH₃ ;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R3: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0, 1, 2 oder 3, wobei die Substituenten R verschieden sein können, wenn o für 2 oder 3 steht; und
- R: C₁-C₄-Alkyl, C₃-C₆-Cycloalkyl, C₁-C₄-Alkoxycarbonyl, Cyano, Aryl und Heteroaryl oder wobei eine CH₂-Gruppe der Alkylenkette durch C=O ersetzt sein kann.

Insbesondere werden außerdem Verbindungen der Formel I.F 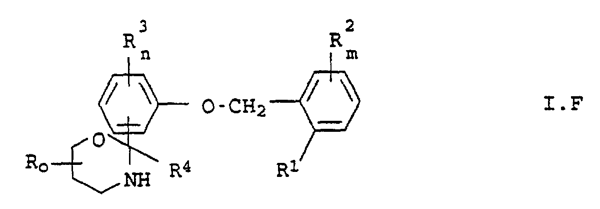 bevorzugt, in denen die Substituenten die folgende Bedeutung haben:
- R¹: C(CO₂CH₃)=CHOCH₃, C (CO₂CH₃)=NOCH₃, N(OCH₃) CO₂CH₃ und C(CONHCH₃)=NOCH₃;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0, 1, 2 oder 3, wobei die Substituenten R verschieden sein können, wenn o für 2 oder 3 steht; und
- R: C₁-C₄-Alkyl, C₃-C₆-Cycloalkyl, C₁-C₄-Alkoxycarbonyl, Cyano, Aryl und Heteroaryl oder wobei eine CH₂-Gruppe der Alkylenkette durch C=O ersetzt sein kann.

Insbesondere werden daneben Verbindungen der Formel I.G 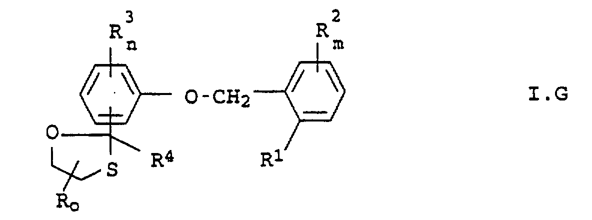 bevorzugt, in denen die Substituenten die folgende Bedeutung haben:
- R¹: C (CO₂CH₃)=CHOCH₃, C (CO₂CH₃)=NOCH₃, N (OCH₃)CO₂CH₃ und C (CONHCH₃)=NOCH₃ ;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0, 1, 3 oder 3, wobei die Substituenten R verschieden sein können, wenn o für 2 oder 3 steht; und
- R: C₁-C₄-Alkyl, C₃-C₆-Cycloalkyl, C₁-C₄-Alkoxycarbonyl, Cyano, Aryl und Heteroaryl oder wobei eine CH₂-Gruppe der Alkylenkette durch C=O ersetzt sein kann.

Insbesondere werden außerdem Verbindungen der Formel I.H 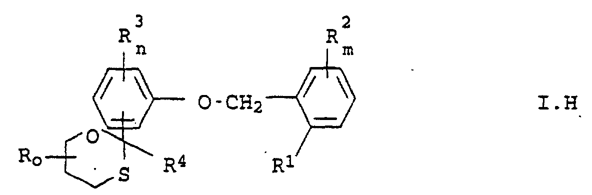 bevorzugt, in denen die Substituenten die folgende Bedeutung haben:
- R¹: C (CO₂CH₃)=CHOCH₃, C (CO₂CH₃)=NOCH₃, N(OCH₃)CO₂CH₃ und C(CONHCH₃)=NOCH₃;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0, 1, 2 oder 3, wobei die Substituenten R verschieden sein können, wenn o für 2 oder 3 steht; und
- R: C₁-C₄-Alkyl, C₃-C₆-Cycloalkyl, C₁-C₄-Alkoxycarbonyl, Aryl und Heteroaryl oder wobei eine CH₂-Gruppe der Alkylenkette durch C=O ersetzt sein kann.

Insbesondere werden daneben Verbindungen der Formel I.K 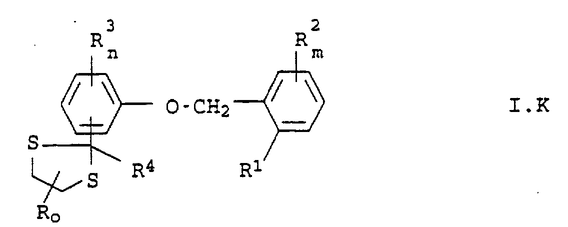 bevorzugt, in denen die Substituenten die folgende Bedeutung haben:
- R¹: C (CO₂CH₃) =CHOCH₃, C(CO₂CH₃) =NOCH₃, N(OCH₃) CO₂CH₃ und C (CONHCH₃) =NOCH₃ ;
- R2: Cyano, Halogen, C₁-C₄-Alkyl und C₁-C₄-Alkoxy;
- m: 0, 1 oder 2, wobei die Substituenten R² verschieden sein können, wenn m für 2 steht;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0, 1, 2 oder 3, wobei die Substituenten R verschieden sein können, wenn o für 2 oder 3 steht; und
- R: C₁-C₄-Alkyl, C₃-C₆-Cycloalkyl, C₁-C₄-Alkoxycarbonyl, Aryl und Heteroaryl oder wobei eine CH₂-Gruppe der Alkylenkette durch C=O ersetzt sein kann.

Insbesondere werden daneben Verbindungen der Formel I.L 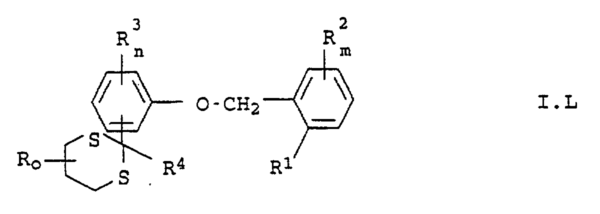 bevorzugt, in denen die Substituenten die folgende Bedeutung haben:
- R¹: C(CO₂CH₃)=CHOCH₃, C(CO₂CH₃)=NOCH₃, N(OCH₃)CO₂CH₃ und C(CONHCH₃)=NOCH₃;
- R²: Cyano, Halogen, C₁-C₄-Alkyl und C₁-C₄-Alkoxy;
- m: 0, 1 oder 2, wobei die Substituenten R² verschieden sein können, wenn m für 2 steht;
- R³: Cyano, Halogen, C₁-C₄-Alkyl, C₁-C₄-Halogenalkyl und C₁-C₄-Alkoxy;
- n: 0, 1, 2 oder 3, wobei die Substituenten R³ verschieden sein können, wenn n für 2 oder 3 steht;
- R⁴: Wasserstoff, C₁-C₄-Alkyl, C₁-C₄-Halogenalkyl, C₃-C₆-Cycloalkyl und Phenyl;
- o: 0, 1, 2 oder 3, wobei die Substituenten R verschieden sein können, wenn o für 2 oder 3 steht; und
- R: C₁-C₄-Alkyl, C₃-C₆-Cycloalkyl, C₁-C₄-Alkoxycarbonyl, Aryl und Heteroaryl oder wobei eine CH₂-Gruppe der Alkylenkette durch C=O ersetzt sein kann.

Insbesondere sind in Hinblick auf ihre Verwendung die in den folgenden Tabellen zusammengestellten Verbindungen I bevorzugt. Die in den Tabellen für einen Substituenten genannten Gruppen stellen außerdem für sich betrachtet (unabhängig von der Kombination, in der sie genannt sind) eine besonders bevorzugte Ausgestaltung des betreffenden Substituenten dar.

### Tabelle 1

Verbindungen der allgemeinen Formel I.1, in denen m=O ist, R³ₙ 2-Methyl bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

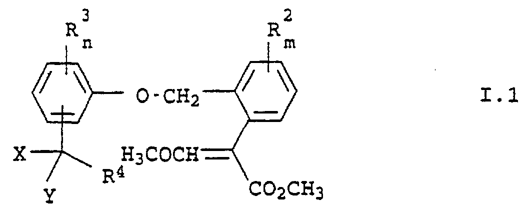

### Tabelle 2

Verbindungen der allgemeinen Formel I.2, in denen m=O ist, R³ₙ 2-Methyl bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

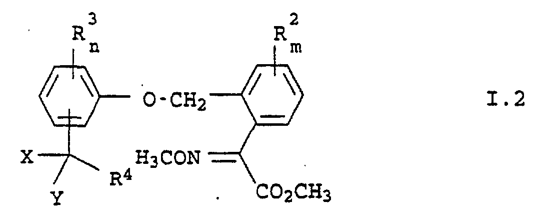

### Tabelle 3

Verbindungen der allgemeinen Formel I.3, in denen m=O ist, R³ₙ 2-Methyl bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

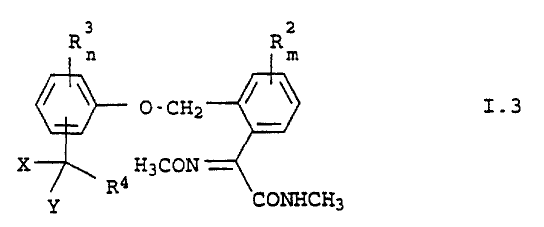

### Tabelle 4

Verbindungen der allgemeinen Formel I.4, in denen m=O ist, R³ₙ 2-Methyl bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

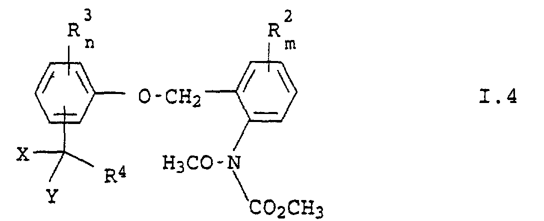

### Tabelle 5

Verbindungen der allgemeinen Formel I.1, in denen m=O ist, R³ₙ 2-Methyl bedeutet und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 6

Verbindungen der allgemeinen Formel I.2, in denen m=O ist, R³ₙ 2-Methyl bedeutet und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 7

Verbindungen der allgemeinen Formel I.3, in denen m=O ist, R³ₙ 2-Methyl bedeutet und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 8

Verbindungen der allgemeinen Formel I.4, in denen m=O ist, R³ₙ 2-Methyl bedeutet und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 9

Verbindungen der allgemeinen Formel I.1, in denen m=O ist, R³ₙ 2-Fluor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 10

Verbindungen der allgemeinen Formel I.2, in denen m=O ist, R³ₙ 2-Fluor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 11

Verbindungen der allgemeinen Formel I.3, in denen m=O ist, R³ₙ 2-Fluor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 12

Verbindungen der allgemeinen Formel I.4, in denen m=O ist, R³ₙ 2-Fluor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 13

Verbindungen der allgemeinen Formel I.1, in denen m=O ist, R³ₙ 2-Fluor bedeutet und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 14

Verbindungen der allgemeinen Formel I.2, in denen m=O ist, R³ₙ 2-Fluor bedeutet und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 15

Verbindungen der allgemeinen Formel I.3, in denen m=O ist, R³ₙ 2-Fluor bedeutet und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 16

Verbindungen der allgemeinen Formel I.4, in denen m=O ist, R³ₙ 2-Fluor bedeutet und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 17

Verbindungen der allgemeinen Formel I.1, in denen m=O ist, R³ₙ 2,5-Dimethyl bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 18

Verbindungen der allgemeinen Formel I.2, in denen m=O ist, R³ₙ 2,5-Dimethyl bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 19

Verbindungen der allgemeinen Formel I.3, in denen m=O ist, R³ₙ 2,5-Dimethyl bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 20

Verbindungen der allgemeinen Formel I.4, in denen m=O ist, R³ₙ 2,5-Dimethyl bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 21

Verbindungen der allgemeinen Formel I.1, in denen m=O ist, R³ₙ 2-Methyl, 5-Chlor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 22

Verbindungen der allgemeinen Formel I.2, in denen m=O ist, R³ₙ 2-Methyl, 5-Chlor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 23

Verbindungen der allgemeinen Formel I.3, in denen m=O ist, R³ₙ 2-Methyl, 5-Chlor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 24

Verbindungen der allgemeinen Formel I.4, in denen m=O ist, R³ₙ 2-Methyl, 5-Chlor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 25

Verbindungen der allgemeinen Formel I.1, in denen m=O ist, R³ₙ 2,5-Dichlor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 26

Verbindungen der allgemeinen Formel I.2, in denen m=O ist, R³ₙ 2,5-Dichlor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 27

Verbindungen der allgemeinen Formel I.3, in denen m=O ist, R³ₙ 2,5-Dichlor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 28

Verbindungen der allgemeinen Formel I.4, in denen M=O ist, R³ₙ 2,5-Dichlor bedeutet und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 29

Verbindungen der allgemeinen Formel I.1, in denen m und n O sind und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 30

Verbindungen der allgemeinen Formel I.2, in denen m und n O sind und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 31

Verbindungen der allgemeinen Formel I.3, in denen m und n O sind und die Kombination der Substituenten X, Y und R⁴ für eine Verbindung jeweils einer Zeile der Tabelle A entspricht, wobei die Gruppe -CXYR⁴ in der Position 4 des Phenylrings gebunden ist.

### Tabelle 32

Verbindungen der allgemeinen Formel I.4, in denen m und n O sind und die Kombination der Substituenten X, Y und R⁴ für eine Verbindung jeweils einer Zeile der Tabelle A entspricht, wobei die Gruppe -CXYR⁴ in der Position 4 des Phenylrings gebunden

### Tabelle 33

Verbindungen der allgemeinen Formel I.1, in denen m und n O sind und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 34

Verbindungen der allgemeinen Formel I.2, in denen m und n O sind und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 35

Verbindungen der allgemeinen Formel I.3, in denen m und n O sind und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

### Tabelle 36

Verbindungen der allgemeinen Formel I.4, in denen m und n O sind und die Kombination der Substituenten X, Y und R⁴ für eine Verbindung jeweils einer Zeile der Tabelle A entspricht, wobei die Gruppe -CXYR⁴ in der Position 5 des Phenylrings gebunden ist.

**Tabelle A**

| | X | Y | R⁴ |
|---|---|---|---|
| 01 | OCH₃ | OCH₃ | H |
| 02 | OCH₃ | OCH₃ | CH₃ |
| 03 | OCH₃ | OCH₃ | CH₂CH₃ |
| 04 | OCH₂CH₃ | OCH₂CH₃ | H |
| 05 | OCH₂CH₃ | OCH₂CH₃ | CH₃ |
| 06 | OCH₂CH₃ | OCH₂CH₃ | CH₂CH₃ |
| 07 | CH₂O | | H |
| 08 | CH₂O | | CH₃ |
| 09 | CH₂O | | CH₂CH₃ |
| 10 | OCH₂CH₂O | | H |
| 11 | OCH₂CH₂O | | CH₃ |
| 12 | OCH₂CH₂O | | CH₂CH₃ |
| 13 | OCH₂CH₂CH₂O | | H |
| 14 | OCH₂CH₂CH₂O | | CH₃ |
| 15 | OCH₂CH₂CH₂O | | CH₂CH₃ |
| 16 | CH(CH₃)O | | H |
| 17 | CH(CH₃)O | | CH₃ |
| 18 | CH(CH₃)O | | CH₂CH₃ |
| 19 | OC(=O)CH₂O | | H |
| 20 | OC(=O)CH₂O | | CH₃ |
| 21 | OC(=O)CH₂O | | CH₂CH₃ |
| 22 | OC(=O)CH₂CH₂O | | H |
| 23 | OC(=O)CH₂CH₂O | | CH₃ |
| 24 | OC(=O)CH₂CH₂O | | CH₂CH₃ |
| 25 | OCH₂CH₂S | | H |
| 26 | OCH₂CH₂S | | CH₃ |
| 27 | OCH₂CH₂S | | CH₂CH₃ |
| 28 | SCH₂CH₂S | | H |
| 29 | SCH₂CH₂S | | CH₃ |
| 30 | SCH₂CH₂S | | CH₂CH₃ |
| 31 | SCH₂CH₂CH₂S | | H |
| 32 | SCH₂CH₂CH₂S | | CH₃ |
| 33 | SCH₂CH₂CH₂S | | CH₂CH₃ |
| 34 | OCH₂CH₂CH₂S | | H |
| 35 | OCH₂CH₂CH₂S | | CH₃ |
| 36 | OCH₂CH₂CH₂S | | CH₂CH₃ |
| 37 | OCH(CH₃)CH₂O | | H |
| 38 | OCH(CH₃)CH₂O | | CH₃ |
| 39 | OCH(CH₃)CH₂O | | CH₂CH₃ |
| 40 | OCH(CH₃)CH(CH₃)O | | H |
| 41 | OCH(CH₃)CH(CH₃)O | | CH₃ |
| 42 | OCH(CH₃)CH(CH₃)O | | CH₂CH₃ |
| 43 | OCH(CH₂CH₃)CH(CH₂CH₃)O | | H |
| 44 | OCH(CH₂CH₃)CH(CH₂CH₃)O | | CH₃ |
| 45 | OCH (CH₂CH₃)CH(CH₂CH₃)O | | CH₂CH₃ |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren und Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindungen gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auchmit Düngemitteln.

Beim Vermischen mit anderen Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,4-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-2,2,2-Trichlor-1-(4-morpholinyl)-ethylformamid, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol; 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino] -acetamid, 1- [2-(2,4-Dichlorphenyl)-pentyl]-1H-1,2,4-triazol, 2,4-Difluor-a-(1H-1,2,4-triazolyl-1-methyl)-benzhydrylalkohol, N-(3-Chlor-2,6-dinitro-4-trifluormethylphenyl) -5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondeasationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
- I.: 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
- II.: 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
- III.: 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
- IV.: 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
- V.: 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
- VI.: Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.%).
- VII.: 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
- VIII.: 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1

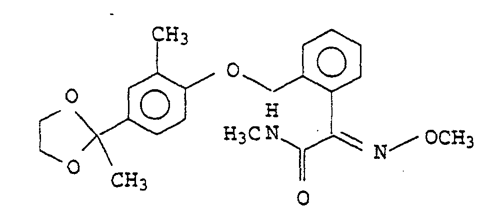

3,5 g 2-[(2'-Methyl-4'-acetyl)-phenoxymethyl]-phenylglyoxylsäuremethylamid-O-methyloxim (Zur Herstellung vgl. DE 43 12 637 A1) werden zusammen mit 0,7 g Ethylenglykol, 1,1 g Ortho-ameisensäure-trimethylester und 1 Tropfen Methansulfonsäure 24 Stunden bei RT (~25°C) gerührt. Zur Aufarbeitung wird mit 0,3 g 30 %-iger NaOCH₃-Lösung im Methanol versetzt, mit Wasser extrahiert und die organische Phase eingeengt. Der Rückstand wird durch Verrühren mit Diisopropylether zur Kristallisation gebracht. Man erhält 2,5 g der Titelverbindung vom Schmelzpunkt 94-97°C.

### Beispiel 2

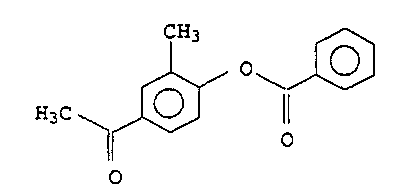

Zu 300,4 g 4-Acetyl-2-methyl-phenol werden 174 g Pyridin und anschließend 309,3 g Benzoylchlorid zugetropft. Nach 1 Stunde bei 40°C wird mit verd. Salzsäure aufgenommen, 1 x mit verd. Salzsäure, 2 x mit NaHCO₃ und 2 x mit Wasser extrahiert, getrocknet und eingeengt. Es bleiben 514,8 g zurück, die man durch Verrühren mit Diisopropylethern zur Kristallisation bringt.
IR (cm⁻¹) = 1734, 1684, 1268, 1129, 699.

### Beispiel 3

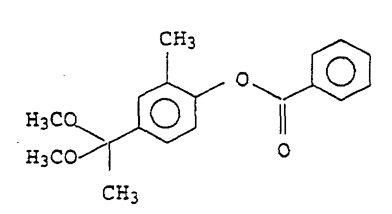

170,4 g der Verbindung aus Beispiel 2 werden in 300 ml Methylenchlorid gelöst und mit 78,2 g Orthoameisensäuretrimethylester sowie 0,2 g Methansulfonsäure versetzt. Nach 120 h bei Raumtemperatur (RT) gibt man weitere 3 g Methansulfonsäure dazu, und rührt weitere 10 h bei RT. Anschließend wird mit 30 %iger NaOCH₃-Lösg. versetzt (pH9), eingeengt, mit tert.-Butyl-methylether aufgenommen, 1 x mit ges. NH₄Cl-Lösg., 3 x mit ges.
NaHCO₃-Lösg. und 2 x mit Wasser gewaschen, getrocknet und eingeengt. Man erhält 226 g Produkt, das ohne weitere Reinigung bei Beispiel 4 eingesetzt wird.

### Beispiel 4

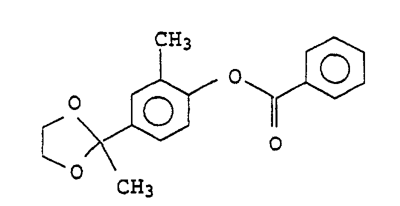

226 g der Verbindung aus Beispiel 3 in 1 Liter Methylenchlorid werden zusammen mit 49 g Ethylenglykol und 6 g Methansulfonsäure 24 Stunden bei Raumtemperatur (RT) gerührt. Man versetzt mit 30 %iger NaOCH₃-Lösg. in Methanol (pH = 9), engt ein, und nimmt den Rückstand in tert.-Butyl-methylether auf. Anschließend wäscht man mit NH₄Cl-Lösg., 3 x mit ges. NaHCO₃-Lösg., 2 x mit Wasser und engt erneut ein. Man erhält 220 g der Titelverbindung als Öl.
IR (cm⁻¹) = 1737, 1267, 1120, 709.

### Beispiel 5

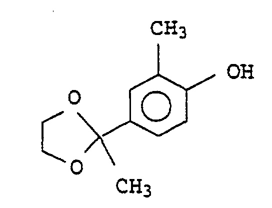

Zu 220 g der Verbindung aus Beispiel 4 werden 52 g KOH in 25 g Wasser zugetropft. Nach 1 Stunde wird eingeengt, in CH₂Cl₂/NH₄Cl-Lösung aufgenommen, 3 x mit Wasser extrahiert, getrocknet und eingeengt. Es bleiben 147 g der Titelverbindung als Öl zurück.
IR (cm⁻¹) = 1274, 1122, 1039, 819.

### Beispiel 6

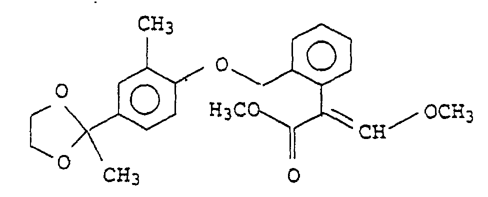

Zu 4,9 g der Verbindung aus Beispiel 5 in 30 ml Dimethylformamid gibt man 5 g NaOCH₃ und 100 mg Kaliumjodid. Nach 1 Stunde tropft man a-(Brommethylphenyl)-β-methoxy-acrylsäuremethylester (zur Herst. vgl. WO 94/05620) in 30 ml DMF und rührt über Nacht bei Raumtemperatur (RT) weiter. Anschließend wird in CH₂Cl₂/Wasser aufgenommen, 3 x mit Wasser extrahiert, getrocknet und eingeengt. Es bleiben 9,7 g zurück, die man über Kieselgel mit Toluol/Aceton (5:1) chromatographiert. Man erhält 1,5 g der Titelverbindung als Öl.
IR (cm⁻¹) = 1709, 1632, 1258, 1131, 770.

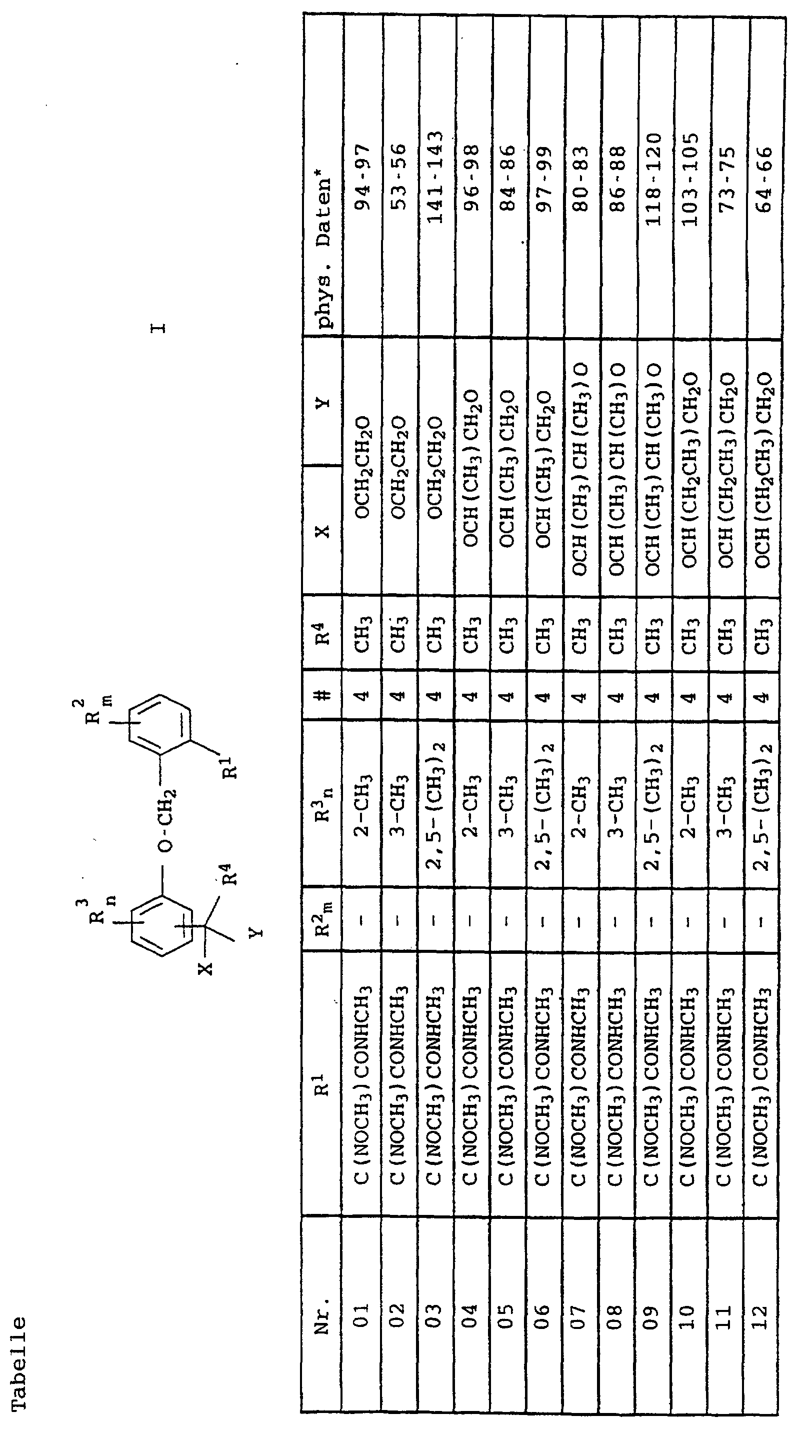

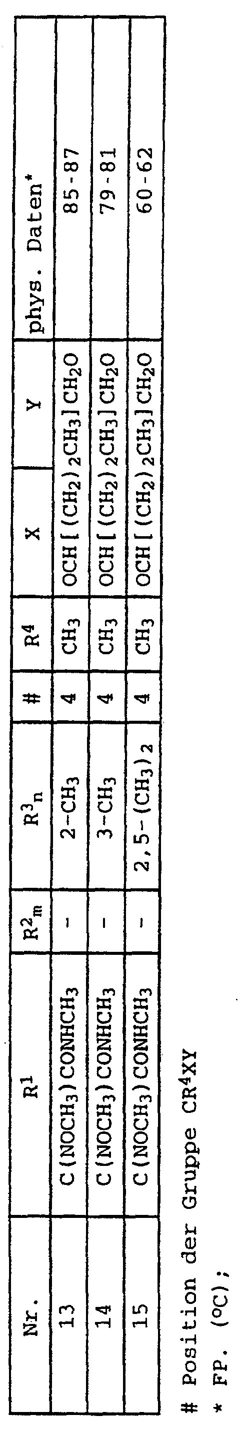

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Fusarium culmorum

Blätter von Weizenkeimlingen (Sorte "Kanzler") wurden zunächst mit der wäßrigen Aufbereitung der Wirkstoffe behandelt (Aufwandmenge: 250 ppm). Nach ca. 24 h wurden die Pflanzen mit einer Sporensuspension des Pilzes *Fusarium culmorum* behandelt. Die so behandelten Pflanzen wurden anschließend für 6 Tage bei 22-24°C und einer relativen Luftfeuchtigkeit von >90% inkubiert. Anschließend wurde das Ausmaß der Pilzentwicklung ermittlelt.

In diesem Test zeigten die mit den erfindungsgemäßen Verbindungen 01, 02 und 04 bis 15 behandelten Pflanzen einen Befall von 15% und weniger, während die unbehandelten (Kontroll-) Pflanzen zu 60% befallen waren.

### Wirkung gegen Pyricularia oryzae (Reisbrand)

Reiskeimlinge (Sorte: "Tai Nong 67") wurden mit der Wirkstoffaufbereitung tropfnaß gespritzt (Aufwandmenge: 63 ppm). Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99% bewahrt. Die Beurteilung erfolgte visuell.

In diesem Test zeigten die mit den erfindungsgemäßen Verbindungen 01 und 03 bis 15 behandelten Pflanzen einen Befall von 15% und weniger, während die unbehandelten (Kontroll-) Pflanzen zu 75% befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

### Die Wirkstoffe wurden

- a.: als 0,1%-ige Lösung in Aceton oder
- b.: als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a. bzw. mit Wasser im Fall von b. verdünnt. Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Verbindungen der allgemeinen Formel I 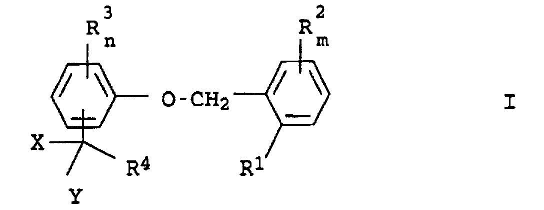 in der die Indices und die Substituenten die folgende Bedeutung haben:
R¹ C(CO₂CH₃)=CHOCH₃, C (CO₂CH₃)=NOCH₃, C (CONHCH₃) =NOCH₃ und N(OCH₃) -CO₂CH₃;
R² Cyano, Halogen, C₁-C₄-Alkyl und C₁-C₄-Alkoxy;
m 0 oder 1;
R³ Cyano, Halogen, C₁-C₄-Alkyl, C₁-C₄-Halogenalkyl und C₁-C₄-Alkoxy;
n 0, 1, 2 oder 3, wobei die Substituenten R³ verschieden sein können, wenn n für 2 oder 3 steht;
R⁴ Wasserstoff, C₁-C₄-Alkyl, C₁-C₄-Halogenalkyl, C₃-C₆-Cycloalkyl und Phenyl;
X, Y C₁-C₆-Alkoxy, C₁-C₆-Alkylthio, C₁-C₆-Alkylamino, Di-C₁-C₆-alkylamino oder gemeinsam eine C₁-C₄-Alkylenkette, welche über Sauerstoff-, Schwefel- und/oder Stickstoff an das C-Atom gebunden ist und welche einen oder zwei der folgenden Substituenten tragen kann: Oxo (=O), Cyano, C₁-C₄-Alkyl, C₃-C₆-Cycloalkyl, C₁-C₄-Alkoxycarbonyl, Aryl und Heteroaryl.

2. Verbindungen der Formel I gemäß Anspruch 1, in denen X und Y gemeinsam mit dem C-Atom, an das sie gebunden sind, einen der folgenden Heterocyclen XY.1 bis XY.9 bilden: 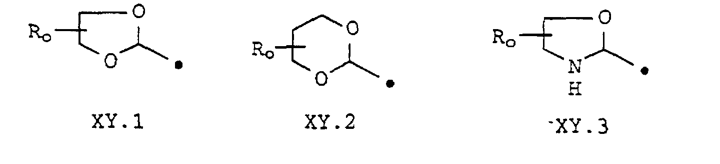 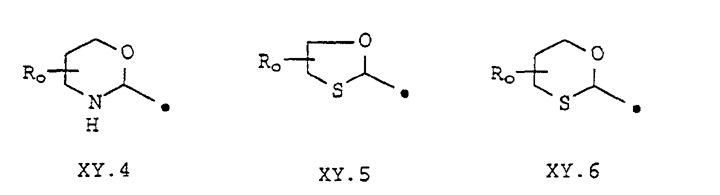 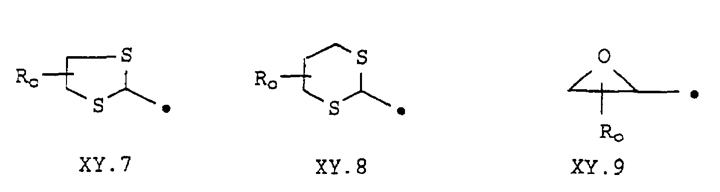 wobei die mit • gekennzeichnete Bindung die Bindung zum Phenylring repräsentiert und wobei
o 0, 1, 2 oder 3 bedeutet, wobei die Substituenten R verschieden sein können, wenn o für 2 oder 3 steht; und
R für C₁-C₄-Alkyl, C₃-C₆-Cycloalkyl, C₁-C₄-Alkoxycarbonyl, Cyano, Aryl und Heteroaryl steht oder wobei eine oder mehrere CH₂-Gruppen der Alkylenkette durch C=O ersetzt sein können.

3. Verfahren zur Herstellung der Verbindungen I gemäß Anspruch 1, in denen die Reste X und Y gemeinsam mit dem C-Atom, an das sie gebunden sind, für einen Oxiranring stehen, **dadurch gekennzeichnet, daß** man eine Carbonylverbindung der Formel II 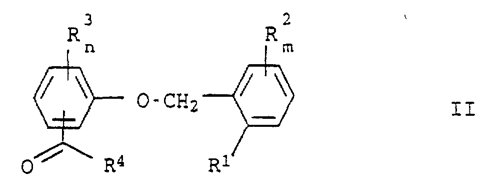 in an sich bekannter Weise in einem inerten organischen Lösungsmittel in Gegenwart einer Base mit einem Trimethylsulfoxonium- oder Trimethylsulfoniumhalogenid umsetzt.

4. Verfahren zur Herstellung der Verbindungen I gemäß Anspruch 1, in denen die Reste X und Y über Sauerstoff-, Schwefel- oder Stickstoff an das C-Atom gebunden sind, **dadurch gekennzeichnet, daß** man eine Carbonylverbindung der Formel II gemäß Anspruch 3 in an sich bekannter Weise in einem inerten organischen Lösungsmittel mit
a) einem Alkohol, Thiol und/oder Amin der Formel III
R^{a}-ZH III
in der
R^{a} C₁-C₆-Alkyl und
Z Sauerstoff, Schwefel, Amino (NH) oder C₁-C₆-Alkylamino bedeutet,
oder mit
b) einer Verbindung der Formel IV
HZ¹-[CₓH₂ₓ₋ₒRₒ] -Z²H IV
in der o und R die in Anspruch 2 gegebene Bedeutung haben,
x 1, 2, 3 oder 4 bedeutet und
Z¹,Z² Sauerstoff, Schwefel, oder Amino (NH) bedeuten,
umsetzt.

5. Verfahren zur Herstellung der Verbindungen I gemäß Anspruch 1, **dadurch gekennzeichnet, daß** man ein Phenol der Formel V 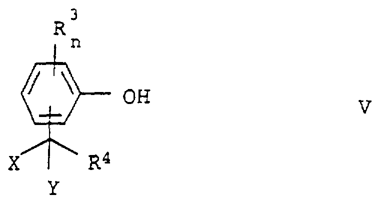 in an sich bekannter Weise in einem inerten organischen Lösungsmittel in Gegenwart einer Base mit einer Benzylverbindung der Formel VI 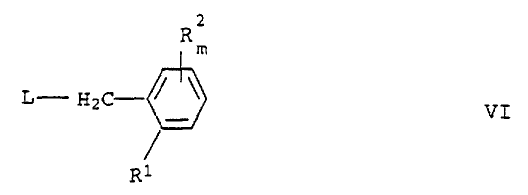 in der L für eine nucleophil austauschbare Gruppe steht, zu I umsetzt.

6. Verfahren zur Herstellung der Verbindungen V gemäß Anspruch 5, in denen X und Y die in Anspruch 4 gegebene Bedeutung haben, **dadurch gekennzeichnet, daß** man einen Phenolester der Formel VII 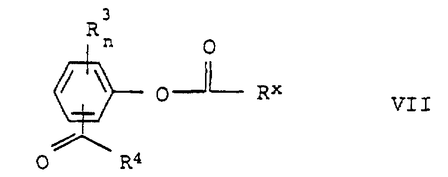 in der R^{x} für Phenyl oder eine Alkylgruppe steht, in an sich bekannter weise in einem inerten organischnen Lösungsmittel mit
a) einem Alkohol, Thiol und/oder Amin der Formel III gemäß Anspruch 4,
oder mit
b) einer Verbindung der Formel IV gemäß Anspruch 4 in den entsprechenden Phenolester VIII 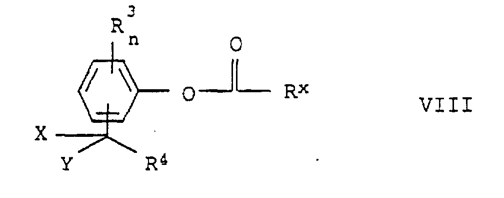 überführt und VIII anschließend in Gegenwart einer Base zu V spaltet.

7. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

8. Verwendung der Verbindungen I gemäß Anspruch 1 zur Herstellung eines zur Bekämpfung von Schädlingen oder Schadpilzen geeigneten Mittels.

9. Verfahren zur Bekämpfung von Schadpilzen, **dadurch gekennzeichnet, daß** man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

10. Verfahren zur Bekämpfung von Schädlingen, **dadurch gekennzeichnet, daß** man die Schädlinge oder die vor ihnen zu schützenden Materialien, Pflanzen, Boden oder Saatgüter mit einer wirksamen Menge einer Verbindung der allgemeinen Formel I gemäß Anspruch 1 behandelt.

## Claims

1. A compound of the general formula I 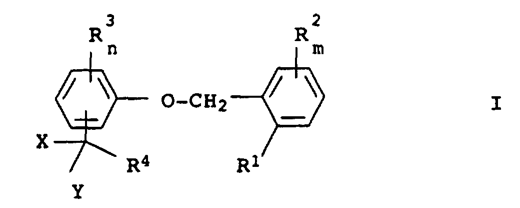 where the indices and the substituents have the following meanings:
R¹ is C(CO₂CH₃)=CHOCH₃, C(CO₂CH₃)=NOCH₃, C(CONHCH₃)=NOCH₃ and N(OCH₃)-CO₂CH₃;
R² is cyano, halogen, C₁-C₄-alkyl and C₁-C₄-alkoxy;
m is 0 or 1;
R³ is cyano, halogen, C₁-C₄-alkyl, C₁-C₄-haloalkyl and C₁-C₄-alkoxy;
n is 0, 1, 2 or 3, it being possible for the substituents R³ to be different from each other when n is 2 or 3;
R⁴ is hydrogen, C₁-C₄-alkyl, C₁-C₄-haloalkyl, C₃-C₆-cycloalkyl and phenyl;
X and Y are C₁-C₆-alkoxy, C₁-C₆-alkylthio, C₁-C₆-alkylamino, di-C₁-C₆-alkylamino or together a C₁-C₄-alkylene chain which is bonded to the C atom via oxygen, sulfur and/or nitrogen and which can have attached to it one or two of the following substituents: oxo (=0), cyano, C₁-C₄-alkyl, C₃-C₆-cycloalkyl, C₁-C₄-alkoxycarbonyl, aryl and heteroaryl.

2. A compound of the formula I as claimed in claim 1, where X and Y together with the C atom to which they are bonded form one of the following heterocycles XY.1 to XY.9: 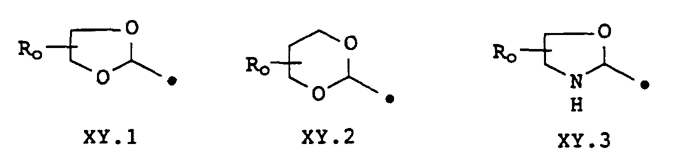 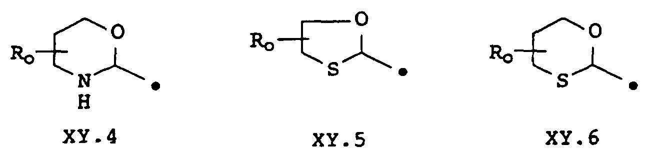 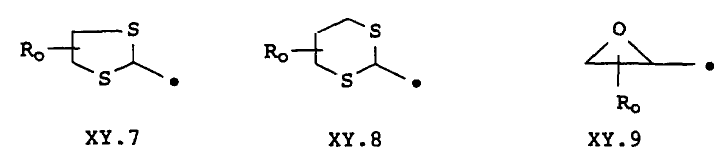 where the bond marked • represents the bond to the phenyl ring and where
o is 0, 1, 2 or 3, it being possible for the substituents R to be different from each other when o is 2 or 3; and
R is C₁-C₄-alkyl, C₃-C₆-cycloalkyl, C₁-C₄-alkoxycarbonyl, cyano, aryl and heteroaryl or it being possible to replace one or more CH₂ groups of the alkylene chain by C=O.

3. A process for the preparation of compounds I as claimed in claim 1 where the radicals X and Y together with the C atom to which they are bonded are an oxirane ring, which comprises reacting a carbonyl compound of the formula II 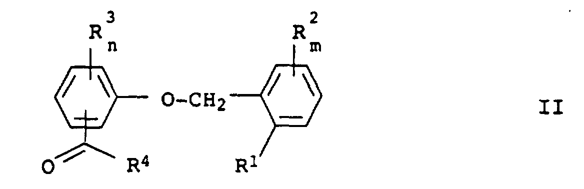 with a trimethylsulfoxonium or trimethylsulfonium halide in a manner known per se in an inert organic solvent in the presence of a base.

4. A process for the preparation of the compounds I as claimed in claim 1 where the radicals X and Y are bonded to the C atom by oxygen, sulfur or nitrogen, which comprises reacting a carbonyl compound of the formula II as claimed in claim 3 with
a) an alcohol, thiol and/or amine of the formula III
R^{a}-ZH III
where
R^{a} is C₁-C₆-alkyl and
Z is oxygen, sulfur, amino (NH) or C₁-C₆-alkylamino,
or with
b) a compound of the formula IV
HZ¹-[CₓH₂ₓ₋ₒRₒ]-Z²H IV
where o and R have the meanings given in claim 2;
x is 1, 2, 3 or 4 and
Z¹ and Z² are oxygen, sulfur or amino (NH),
in a manner known per se in an inert organic solvent.

5. A process for the preparation of the compounds I as claimed in claim 1, which comprises reacting a phenol of the formula V 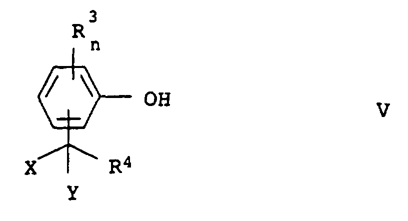 with a benzyl compound of the formula VI 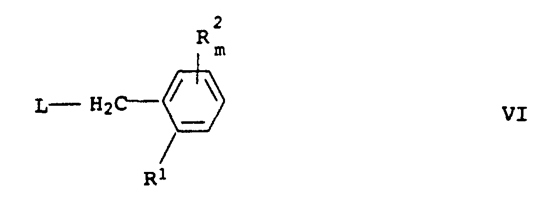 where L is a nucleophilically exchangeable group
in a manner known per se in an inert organic solvent in the presence of a base to give I.

6. A process for the preparation of the compounds V as claimed in claim 5 where X and Y have the meanings given in claim 4, which comprises converting a phenol ester of the formula VII 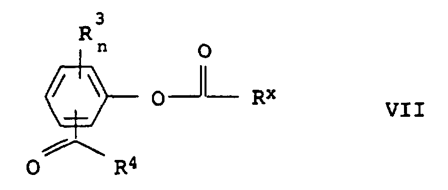 where R^{x} is a phenyl or an alkyl group with
a) an alcohol, thiol and/or amine of the formula III as claimed in claim 4
or with
b) a compound of the formula IV as claimed in claim 4
in a manner known per se in an inert organic solvent to give the corresponding phenol ester VIII 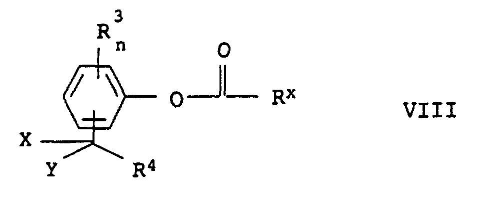 and subsequently cleaving VIII in the presence of a base to give V.

7. A composition which is suitable for controlling pests or harmful fungi, comprising a solid or liquid carrier and a compound of the general formula I as claimed in claim 1.

8. The use of the compounds I as claimed in claim 1 for the preparation of a composition which is suitable for controlling pests or harmful fungi.

9. A method of controlling harmful fungi, which comprises treating the fungi, or the materials, plants, soils or seed to be protected against fungal infection with an effective amount of a compound of the general formula I as claimed in claim 1.

10. A method of controlling pests, which comprises treating the pests or the materials, plants, soils or seeds to be protected against them with an effective amount of a compound of the general formula I as claimed in claim 1.

## Revendications

1. Composés de formule générale I 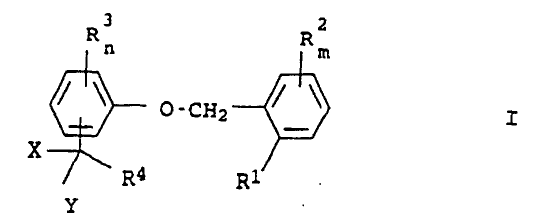 dans laquelle les indices et les symboles ont les significations suivantes :
R¹ : C(CO₂CH₃)=CHOCH₃, C(CO₂CH₃)=NOCH₃,
C(CONHCH₃)=NOCH₃ ou N(OCH₃)-CO₂CH₃ ;
R² : un groupe cyano, un halogène, un groupe alkyle en C1-C4 ou alcoxy en C1-C4 ;
m : 0 ou 1 ;
R³ : un groupe cyano, un halogène, un groupe alkyle en C1-C4, halogénoalkyle en C1-C4 ou alcoxy en C1-C4 ;
n : 0, 1, 2 ou 3, les substituants R³ pouvant être différents lorsque n est égal à 2 ou 3 ;
R⁴ : l'hydrogène, un groupe alkyle en C1-C4, halogénoalkyle en C1-C4, cycloalkyle en C3-C6 ou phényle ;
X, Y : des groupes alcoxy en C1-C6, alkylthio en C1-C6, alkylamino en C1-C6, di-(alkyle en C1-C6)amino ou bien, ensemble, une chaîne alkylène en C1-C4 qui peut être reliée à l'atome de carbone par l'intermédiaire d'un atome d'oxygène, de soufre et/ou d'azote et qui peut porter un ou deux des substituants suivants : oxo (=O), cyano, alkyle en C1-C4, cycloalkyle en C3-C6, (alcoxy en C1-C4)carbonyle, aryle et hétéroaryle.

2. Composés de formule I selon la revendication 1, dans laquelle X et Y forment ensemble et avec l'atome de carbone auquel ils sont reliés l'un des hétérocycles suivants XY.1 à XY.9 : 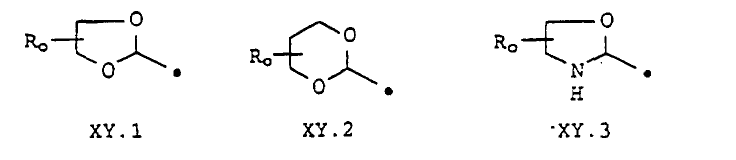 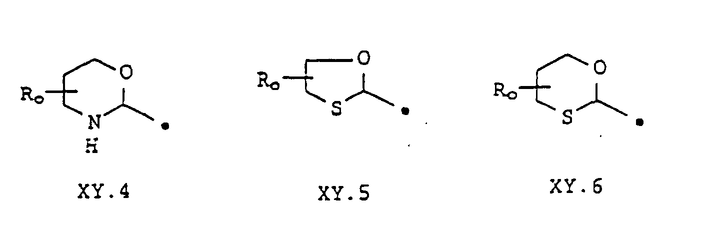 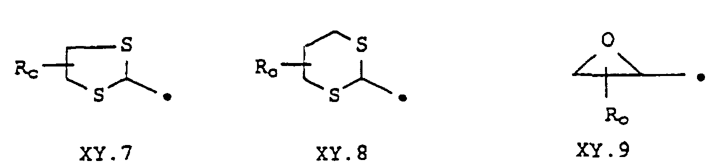 le signe • indiquant la liaison au cycle phényle et
o est égal à 0, 1, 2 ou 3, les substituants R pouvant être différents lorsque o est égal à 2 ou 3 ; et
R représente un groupe alkyle en C1-C4, cycloalkyle en C3-C6, (alcoxy en C1-C4)carbonyle, cyano, aryle ou hétéroaryle, un ou plusieurs groupes CH₂ de la chaîne alkylène pouvant être remplacée par C=O.

3. Procédé pour la préparation des composés I de la revendication 1 pour lesquels X et Y représentent ensemble et avec l'atome de carbone auquel ils sont reliés un cycle oxirane, **caractérisé par le fait que** l'on fait réagir un dérivé carbonylé de formule II 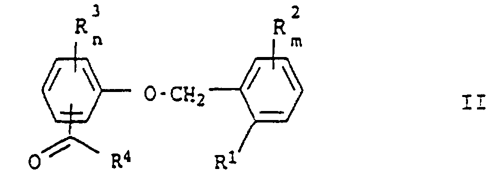 de manière connue en soi, dans un solvant organique inerte et en présence d'une base, avec un halogénure de triméthylsulfoxonium ou de triméthylsulfonium.

4. Procédé pour la préparation des composés I de la revendication 1 pour lesquels X et Y sont reliés à l'atome de carbone par l'intermédiaire de l'oxygène, du soufre ou de l'azote, **caractérisé par le fait que** l'on fait réagir un dérivé carbonylé de formule II de la revendication 3, de manière connue en soi, dans un solvant organique inerte, avec
a) un alcool, un thiol et/ou une amine de formule III
R^{a}-ZH III
dans laquelle
R^{a} représente un groupe alkyle en C1-C6 et
Z représente l'oxygène, le soufre, un groupe amino (NH) ou alkylamino en C1-C6, ou bien avec
b) un composé de formule IV
HZ¹-[CₓH₂ₓ-ₒRₒ]-Z²H IV
dans laquelle o et R ont les significations indiquées dans la revendication 2,
x est égal à 1, 2, 3 ou 4 est
Z¹, Z² représentent chacun l'oxygène, le soufre ou un groupe amino (NH).

5. Procédé pour la préparation des composés I de la revendication 1, **caractérisé par le fait que** l'on fait réagir un phénol de formule V 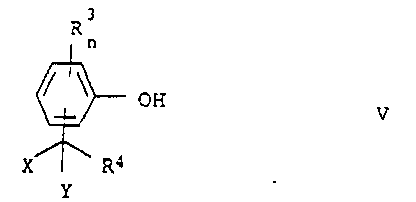 de manière connue en soi, dans un solvant organique inerte et en présence d'une base, avec un dérivé benzylique de formule VI 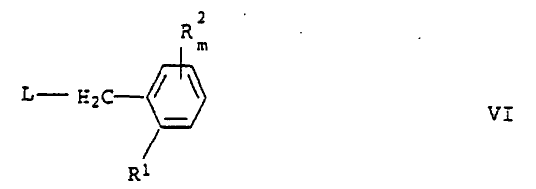 dans laquelle L représente un groupe apte à un échange nucléophile, ce qui donne un composé I.

6. Procédé pour la préparation des composés V selon la revendication 5 pour lesquels X et Y ont les significations indiquées dans la revendication 4, **caractérisé par le fait que** l'on convertit un ester de phénol de formule VII 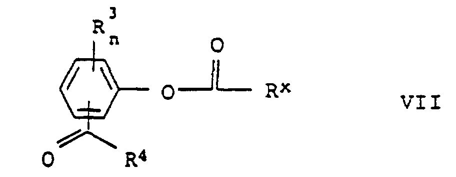 dans laquelle R^{x} représente un groupe phényle ou alkyle, de manière connue en soi, dans un solvant organique inerte, par réaction avec
a) un alcool, un thiol et/ou une amine de formule III de la revendication 4,
ou avec
b) un composé de formule IV de la revendication 4, en l'ester de phénol correspondant VIII 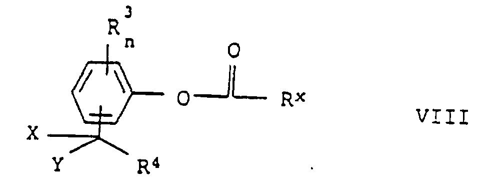 qu'on scinde ensuite en V en présence d'une base.

7. Produit convenant à l'utilisation pour la lutte contre les parasites ou les mycètes nuisibles, qui contient un véhicule solide ou liquide et un composé de formule générale I de la revendication 1.

8. Utilisation des composés I de la revendication 1 pour la préparation d'un produit convenant à l'utilisation pour la lutte contre les parasites ou les mycètes nuisibles.

9. Procédé pour combattre les mycètes nuisibles, **caractérisé par le fait que** l'on traite les mycètes ou les matériaux, végétaux, sols ou semences à protéger contre une attaque par les mycètes, par une quantité efficace d'un composé de formule générale I de la revendication 1.

10. Procédé pour combattre les parasites, **caractérisé par le fait que** l'on traite les parasites ou les matériaux, végétaux, sols ou semences qu'on veut protéger contre les parasites par une quantité efficace d'un composé de formule I selon la revendication 1.
